Europäisches Patentamt

(19) European Patent Office

Office européen des brevets

(11) Publication number: **0 352 802**
**A2**

## (12) EUROPEAN PATENT APPLICATION

(21) Application number: 89113910.7

(22) Date of filing: 27.07.89

(51) Int. Cl.⁴: **C08J 5/18 , C08L 23/02 , C08K 3/00**

(30) Priority: 27.07.88 US 224632

(43) Date of publication of application:
**31.01.90 Bulletin 90/05**

(84) Designated Contracting States:
**BE CH DE ES FR GB IT LI LU NL SE**

(71) Applicant: **HERCULES INCORPORATED**
**Hercules Plaza**
**Wilmington Delaware 19894(US)**

(72) Inventor: **Antoon, Mitchell Kelly**
**2405 Dacia Drive Lancashire**
**Wilmington Delaware 19810(US)**
Inventor: **Hill, David James**
**2200 Jamaica Drive Holiday Hills**
**Wilmington Delaware 19810(US)**

(74) Representative: **Lederer, Franz, Dr. et al**
**Van der Werth, Lederer & Riederer**
**Patentanwälte Lucile-Grahn-Strasse 22**
**D-8000 München 80(DE)**

(54) **Breathable microporous film and method for making it.**

(57) A method for making soft, flexible, microporous films having high tensile strength and high breathability levels for air and water vapor and high hydrostatic resistance to penetration by liquid water, in which a film fabricated by blending a mixture of a polymer or copolymer of an alpha-olefin with 60 to 75% by weight of an inorganic filler or glass beads having particle sizes within the range of 10 to 15 micrometers and calcium stearate as a processing aid is biaxially stretched from about 1.5 to about 7 times in each direction at a temperature of from about 20° to about 160°C, and the moisture level in the blended composition is maintained below 700 parts per million before fabricating the film; the film so made having a Gurley porosity of from 0.1 to 20 seconds; and its use in disposable items such as panty liners, diapers, bed sheets, and hospital gowns, or, if the filler consists of glass beads, its use as a battery separator.

EP 0 352 802 A2

## BREATHABLE MICROPOROUS FILM AND METHOD FOR MAKING IT

This invention relates to methods for making soft, flexible, microporous films having high tensile strength and good permeance or "breathability" levels for air and water vapor and high hydrostatic resistance to penetration by liquid water, to films made by the said methods, and to the uses of such films.

It is well known that thermoplastic polymers can be filled with inert fillers, cast into sheets, and stretched to form an oriented microporous thermoplastic film that provides a desired level of gas or vapor permeance. Methods for making such films are described, for example, in U.S. Patents 3,903,234 and 4,698,372, in UK Patent Specification 2,151,538, and in European published Patent Application 272,026, the latter disclosing films of homopolymers, copolymers, or blends of alpha-monoolefins having 2 to 10 carbons having an oxygen and carbon dioxide permeance between about 77,500 and 155,000,000 cc/m$^2$-day-atmosphere.

U.S. Patent No. 4,698,372 discloses microporous polymeric films for use as substitutes for textiles and having good water vapor transmission rates and hydrostatic resistance to water penetration; the films may consist of certain ethylene copolymers and have a filler loading of about 25-35 volume % of inorganic fillers such as calcium carbonate; they use a processing aid such as stearic acid that is referred to as an "antagonizer" and is said to reduce the effective surface tension of the filler to the approximate level of that of the matrix polymer. U.S. Patent No. 3,903,234 discloses gas-permeable biaxially oriented film prepared from $C_2$-$C_{10}$ alpha-monoolefin polymers containing 26-50% by weight of inorganic filler particles. UK Patent Specification 2,151,538 discloses a method for making water-vapor-permeable oriented films from polyolefins containing 33-83% by weight of barium sulfate filler, for use as a leakproof sheet in disposable diapers and sanitary napkins.

In general, the available liquid-barrier films that have an appreciable level of "breathability", for instance, those sold as diaper liners or covers for panty liners, do not reach desirable levels of breathability. This can be evaluated quantitatively in term of Gurley porosity numbers, which are measured in seconds by ASTM D-726, Method A or Method B. (Theoretically, Gurley numbers measured by Method A are 25 times larger than Gurley numbers measured by Method B, and Method B numbers will be used in the following description and claims). Method B of ASTM D-726 measures the time (in seconds) for ten milliliters of air to pass through one square inch of microporous film under a pressure of 12.2 inches of water. (Method A of ASTM D-726 measures the time (in seconds) for 100 milliliters of air to pass through one square inch of microporous film under a pressure of 4.9 inches of water). A low Gurley number signifies that a microporous film offers little resistance to the passage of air (or humid air). Thus, Gurley numbers, also known as Gurley porosity numbers, are effective measures of "breathability".

The generally available films have Gurley numbers up to 100 seconds and usually above 10 to 20 seconds. The unavailability of soft, flexible, microporous liquid-barrier films having high tensile strength and a higher level of "breathability", at a reasonable cost, indicates that there is a need, particularly in the hygienic product industry, for such films. They are needed, for example, in the fabrication of disposable products such as feminine panty liners, diapers, bed sheets, and hospital gowns that are cool and comfortable for the wearer. There is also a need for such permeable films, which have an ion-exchange function, for use as battery separators.

According to the invention, a method for making soft, flexible, microporous films having high tensile strength and good permeance or "breathability" levels for air and water vapor and high hydrostatic resistance to penetration by liquid water, including the steps of melt-blending a mixture of a polymer or copolymer of an alpha-olefin, a particulate filler, and calcium stearate as a processing aid, fabricating a film, and biaxially stretching the film, is characterized in that the mixture contains 60 to 75% by weight of an inorganic filler or glass beads having particle sizes within the range of 10 to 15 micrometers in mean diameter, the moisture level in the blended composition is maintained below 700 ppm prior to fabricating the film, and the film is stretched in two directions from about 1.5 to about 7 times in each direction at a temperature range of from about 20° to about 160° C.

Unless the moisture level in the composition prior to fabricating the film is maintained below 700 ppm, the film cannot be stretched uniformly, using the amount of filler required according to the invention. Preferably, the moisture level in the blended composition is maintained below 300 ppm.

Also according to the invention, a soft, flexible, microporous film having high tensile strength and good permeance or "breathability" levels for air and water vapor and high hydrostatic resistance to penetration by liquid water, and made by the said method according to the invention, is further characterized in that it comprises:

20 to 37% by weight of a polymer or copolymer of an alpha-olefin having 1-8 carbon atoms,

60 to 75% by weight of an inorganic filler or glass beads having particle sizes within the range of of 10 to 15 micrometers in mean diameter, and

0.1 to 3% by weight of calcium stearate, and has a Gurley porosity, based on method B, ASTM D-726, of 0.1 second to 20 seconds.

Preferably the film according to the invention, contains 0 to 2% by weight of a stabilizer.

"Microporous" means that the film contains numerous open pores or channels leading from one surface to the opposite surface, such pores being of a size to permit air and water vapor to pass through the film while having good resistance to the penetration of liquid water. Their porosity or breathability in Gurley porosity numbers can be expressed in terms of permeance by dividing the constant $44.64 \times 10^9$ seconds by the Gurley number (Method B) to give the permeance in cc/m²-day-atmosphere. The permeance of the microporous film of this invention is greater than $44.64 \times 10^9$ seconds- cc/m²-day-atmosphere divided by 20 seconds, namely $2.232 \times 10^9$ or 2,232,000,000 cc/m²-day-atmosphere.

The particle size of the filler determines the pore size of the microporous films of this invention. As would be expected, smaller particle sizes produce smaller pores than larger particle sizes. There is no theoretical limitation on the size of the filler particles that may be used in the practice of this invention. However, practical considerations impose effective limitations. Preferably, the particle size of the fillers should range from 10 to 25 micrometers in mean diameter, and preferably the filler is calcium carbonate and the particle size is about 12.5 micrometers in mean diameter.

Filler-loading determines to a great extent how far the casting must be stretched to attain a given degree of overall porosity. Below the lower end of the loading range, 60% by weight, where the pores are less numerous, they are less interconnected, and the film is insufficiently permeable at the maximum draw ratio of about 7 times in each direction according to the invention. Above the higher end of the loading range, 75% by weight, either the materials will not blend uniformly or the casting made from the composition will not stretch the minimum according to the invention, namely, 1.5 times. Although other inorganic fillers may be used, calcium carbonate is preferred. However, if the films are to be used as battery separators, glass beads are used as the filler.

The calcium stearate processing aid coats the filler particles, thus assisting in the uniform dispersion of the filler particles, and allowing the composition to be stretched to the required degree of orientation.

A variety of alpha-olefinic polymeric materials having 1-8 carbon atoms can be used as matrix polymers. Any such alpha-olefin that exhibits sufficient tensile yielding and some permanent deformation may be used. The selection of the polymeric material will be based on the desired properties of the microporous film, as for example, temperature resistance or elastic recovery. Preferred homopolymers are polypropylene (PP), polyethylene (PE) (particularly linear low-density polyethylene (LLDPE)), and polybutylene (PB). Copolymers of ethylene with propylene or with an alpha-olefin of 4-8 carbon atoms may of course be used also. For ease in processing, a blend of linear low density polyethylene and polypropylene in a ratio of 95 to 5, or polypropylene blended with ethylene- propylene copolymer, is preferable to polypropylene alone as a matrix material.

The choice of the polymeric material in the matrix, and the choice of the filler-material influences the prefered amounts of the filler and the calcium stearate, the orientation temperature, and the extent of biaxial orientation.

With a polypropylene/calcium carbonate combination, the mixture preferably contains about 33% by weight of polypropylene, and the amount of the calcium carbonate filler preferably is about 65% by weight. The amount of calcium stearate preferably is about 0.5 to about 2.0%, and more preferably 2%. The biaxial orientation of the film preferably is in the range of from about 4 to about 7 times in each direction, more preferably about 5 times, with the orientation temperature preferably being from 130 to 150°C, with about 130°C being most preferred.

With a polypropylene/glass beads combination, the mixture preferably contains about 33.5% by weight of polypropylene, the amount of the glass beads filler preferably being in the range of 55 to 65% by weight, more preferably 65%. The amount of calcium stearate preferably is about 0.5 to about 2.0%, and most preferably 1.5%. The biaxial orientation of the film preferably is in the range of from about 4 to about 7 times in each direction, more preferably about 5 times, with the orientation temperature preferably being from 130 to 150°C, with about 135°C being most preferred.

With a polybutylene/calcium carbonate combination, the mixture preferably contains about 65 to about 75% by weight of the filler, with about 70% being more preferred; in this combination the amount of calcium stearate to be used should be in the range of about 0.2 to about 4% by weight, with about 2% being more preferred. This film should be biaxbiaxially oriented from about 1.5 to about 5 times, more preferably about 4 times, at a temperature range of about 20 to about 105°C, about 100°C being more preferred.

With a polyethylene/calcium carbonate combination, the amount of filler preferably is in the range of about 60 to about 70% by weight, about 70% being more preferred. The amount of calcium stearate in this combination preferably is from about 0.1 to about 3.5%, about 0.5 to about 2% being more preferred. This film preferably is biaxially oriented about 1.5 to about 5 times, with about 4 times being more preferred, in a temperature range of about 20°C to about 110°C, with about 100°C being more preferred.

Preferably the mixture contains from about 0.1 to about 2% by weight of a stabilizer against degradation by exposure to UV light, oxygen, and heat; it is especially useful in the combinations with the polypropylene and the polyethylene.

After the film composition is prepared, it may be compounded into the film of this invention by any known method suitable for the melt blending of thermoplastic polymers at temperatures at which the matrix polymers are processible. High shear mixing, which can be achieved in a Banbury-type or another high intensity mixer or in continuous mixers such as extruders, is preferred. There is no need to premix ingredients, but this may be done without detriment to the practice of this invention and may in certain instances offer improved performance.

After the ingredients of the composition of this invention have been melt blended, the moisture level of this blend is then maintained below the critical level of 700 parts per million (ppm) (more preferably below 300 ppm), preferably by simultaneously cooling and maintaining the moisture level of the blended and extruded composition by flowing air over it on a moving conveyor belt. This air-cooling method yields strands and pellets that have residual moisture levels far below the levels achieved by the process of cooling by immersion in a water bath that is conventionally used.

The strands were then pelletized using conventional techniques. To accurately achieve this moisture level, sensitive moisture measurement is required. For example, a Coulometric Karl Fischer titration method (using the Brinkman Model 652 RF Coulometer) can be used for evaluating the moisture level in the formulations.

After blending and establishing the moisture level, the composition is converted into any convenient form for processing into film, including pellets or sheets. The film fabrication can be accomplished by any conventional technique including extrusion casting, compression molding, flat film extrusion, or blown film extrusion.

After the film is fabricated into its desired form, it is then biaxially oriented by stretching by any of the well known techniques in the art including, by hydraulics, by pinch rolls moving at different rates, or by tentering. Biaxial stretching can be performed sequentially or simultaneously. Sequential biaxial stretching is preferred when using the tentering operation.

Another process for maintaining the desired moisture level is to employ vacuum-drying in order to reduce the moisture level in too-wet pellets to acceptable levels according to the invention, (below 700 ppm, and more preferably below 300 ppm). In this case, pellets composed of polymer plus filler are made using the conventional water-bath-cooling process, which produces is excessive residual moisture levels. These too-wet pellets are subjected to a partial vacuum, preferably with some heating to speed the process, for a period of time until the moisture content is within acceptable limits as defined above. This process is not preferred since the extra step of vacuum-drying is required.

Yet another process of maintaining the desired moisture level is by charging the hot melt directly to the extruder that extrudes the casting from a die. In this case, the molten composition is never exposed to water and has a low residual moisture level as defined above. Therefore, a smooth and highly-orientable casting will be formed.

The stretch ratio of at least two times the original forming dimensions is significant to producing a film having at least 30% of pores resulting in relatively high density films. However, to produce relatively low density films, it is preferred that the film be stretched to at least 3 to 8 times its original forming dimensions in mutually perpendicular directions, resulting in a film having about 40 to 70% pores.

Stretching is of course effected above the glass transition temperature of the matrix polymer (preferably at least 20°C above) and below the melting temperature of the matrix polymer, especially within 10°C of that temperature, depending to some degree on the rate of stretching. Different polymers and compositions thereof exhibit different elastic and viscoelastic behavior. Thus, different amounts of stretching must be imposed on different samples in order to obtain the same permeability properties. Obviously, the film must be stretched beyond its yield point in order to attain the permanent deformation necessary for the formation of porosity.

For a given composition, a greater degree of stretch results in greater overall porosity. Higher overall porosity can be attained by adding more filler and stretching the same amount or possibly less.

In the following examples, all parts, proportions, and percentages are by weight unless otherwise indicated.

In Examples 1-8, the ingredients (listed in Table 1A) were blended at room temperature and compounded in a twin-screw extruder; strands were extruded at in a temperature range of 243° to 265° C. The strands were then air cooled (except that in Examples 6-8 they were water cooled) and pelletized. The pellets were vacuum dried for 24 hours at 80° C. (except that in Examples 7 and 8 they were vacuum dried for 8 hours at 70° C.). Using a melt temperature of 278 to 282° C (478 to 540° F), the pellets were extruded by a single screw extruder through a six inch wide slit die onto a casting roll maintained at about 65° C (except Examples 6-8 were maintained at about 18-24° C) so as to form a 15 mil thick casting. Using a T.M. Long stretcher, square pieces having the dimensions 2 x 2 inches from the casting were biaxially oriented by stretching 4 times in the machine direction and 4 times in the transverse direction (except that in Example 8 it was stretched 2x by 2x) at 100° C, producing the product as set forth in the following Table 1.

The ingredients for Examples 9-14 are listed in Table 1B. In Examples 9 and 10, the ingredients were blended together on a 2-roll mill at 200° C.; this blend was compression molded at 215° C. to yield 30 mil thick plaques. Two inch by two inch portions of the plaques were biaxially oriented by stretching 5 times in the machine direction and 5 times in the transverse direction on a T. M. Long stretcher at 140° C. to make the film as described in Table 1B.

In Examples 11 and 14, the ingredients were compounded in a twin screw extruder at 225-250° C.; the extrudate was pelletized and cast on a casting extruder at 180°-230° C. For Example 11 since much strand breakage and non-uniformity was observed during the pelletizing step, the casting could not be stretched at 140° C. on the T. M. Long stretcher; the casting was too brittle. For Example 14, 2 X 2 inch portions of the casting were stretched 4.5X by 4.5X at 140° C. on the T. M. Long stretcher.

In Example 12 and 13, the ingredients were blended by a twin-screw extruder and were extruded by a single screw extruder and slit die to form a 30 mil casting; the casting was stretched 5X by 5X on a T. M. Long stretcher to form the film.

EP 0 352 802 A2

TABLE 1A

|  | 1a.b.c. | 2 | 3 | Com 4 | 5 | 6 | Com 7 | Com 8 |
|---|---|---|---|---|---|---|---|---|
| POLYMER | LLDPE | LLDPE | LLDPE | LLDPE | LLDPE/PP | PB | PB | PB |
| (%) | 28.5 | 33.25 | 38.0 | 29.4 | 27.0/1.5 | 28.5 | 50.0 | 30.0 |
| CALCIUM STEARATE (%) | 1.5 | 1.75 | 2.0 | 0.6 | 1.5 | 1.5 | 0 | 0 |
| CaCO,(%) | 70 | 65 | 60 | 70 | 70 | 70 | 50 | 70 |
| STABILIZER |  |  |  |  |  |  |  |  |
| GLASS BEADS (%) |  |  |  |  |  |  |  |  |
| **PRODUCT PROPERTIES** | | | | | | | | |
|  | 1a.b.c. | 2 | 3 | Com 4 | 5 | 6 | Com 7 | Com 8 |
| ORIENTATION/TEMP°C | a 4xby4x/100°C<br>b 3xby3x/100°C<br>c 2xby2x/100°C | 4xby4x/100°C | 4xby4x/100°C | Mat'ls. would not mix well | 4xby4x/100°C | 4xby4x/100°C | 4xby4x/100°C | 2xby2x/100° |
| FILM THICKNESS (mil) | a = 2.5<br>b = 6.0<br>c = 10.5 | 3.0 | 3.5 |  | 3.0 | 4.0 | 3.0 |  |
| % AIR VOIDS | a = 65<br>b = 65<br>c = 65 | 66 | 61 |  |  | 60 |  |  |
| Gurley POROSITY (METHOD B) | a = 0.1sec<br>b = 0.2sec<br>c = 0.4sec | 0.5sec | 0.8sec |  | 0.3sec | 1.4sec | 30sec |  |

*C$_2$C$_3$ is an ethylene-propylene copolymer containing 2.7 mole percent ethylene units

TABLE 1 B

|  | 9 | Com 10 | 11 | 12 | 13 | 14 |
|---|---|---|---|---|---|---|
| POLYMER (%) | PP/C$_2$C$_3$* (1:1) 39.83 | PP/C$_2$C$_3$ (1:1) 49.74 | PP 39.64 | PP/C$_2$C$_3$ 34.61 | PP/C$_2$C$_3$ (1:1) 34.91 | PP (1:1) 36.05 |
| CALCIUM STEARATE (%) | 0.32 | 0.40 | 0.40 | 1.04 | 0.17 | 1.09 |
| CaCO$_3$(‰) | 59.75 | 49.74 | 59.46 | 64.27 | 64.83 | - |
| STABILIZER | 0.10 | 0.12 | 0.50 | 0.09 | 0.09 | 0.16 |
| GLASS BEADS (%) |  |  |  |  |  | 62.71 |
| PRODUCT PROPERTIES | | | | | | |
|  | 9 | Com 10 | 11 | 12 | 13 | 14 |
| ORIENTATION/TEMP °C | 5xby5x/140°C | 5xby5x/140°C | would not stretch 140°C | 5xby5x | 5xby5x | 4.5xby4.5x/140°C |
| FILM THICKNESS (mil) |  |  |  |  |  |  |
| % AIR VOIDS |  |  |  |  |  |  |
| Gurley POROSITY (METHOD B) | 85sec | 10min |  | 20sec |  | 4.0sec. |

*C$_2$C$_3$ is an ethylene-propylene copolymer containing 2.7 mole percent ethylene units.

All of the resulting products of the Examples were opaque white films.

Examples 1a, b, and c show that for the system LLDPE/CaCO$_3$ films, 70% by weight of the filler gives much lower Gurley number (i.e., high breathability) than 65% by weight of the filler. Examples 1a, b and c show that breathability is the best in the more highly oriented films. Similarly, 65% by weight of the filler (Example 2) gives a much lower Gurley number than 60% filler, (Example 3). Example 4 compared to Example 1 shows that the processibility of the formulation is improved by adding 1.5% of calcium stearate (Example 1) instead of 0.6% calcium stearate (Example 4); further, in comparative Example 4, die deposits and melt fractures were excessive and caused constant breakage of the extruded molten strands. Thus, the material could not be pelletized and extruded into castings suitable for orientation. Example 5 demonstrates the advantage of using a small amount of polypropylene additive in the LLDPE to reduce die lines. Regions of melt fracture (die lines) thinner than the rest of the casting were greatly reduced compared to the melt fracture regions commonly observed in compositions such as those in Examples 1a, b and c.

Example 6 shows that, for polybutene/calcium carbonate film, 70% by weight of the filler gives much lower Gurley number (i.e., high breathability) than 50% by weight of the filler in Example 7. Example 8, compared to Example 6, shows that 1.5% of calcium stearate allows much easier processing to a porous film than if no calcium stearate is used (Example 8). The film prepared in comparative Example 8 had many visible pinholes and was extremely rough. Gurley measurements were not possible. Castings made from this composition could not be oriented 4 times by 4 times at the temperature of 100°C. Example 9 shows that, for polypropylene/calcium carbonate films, 60% by weight of the filler gives a much lower Gurley number than when only 50% by weight of the filler is used (Example 10). Example 11 compared to Example 9, shows that using a blend of polypropylene and ethylene-propylene copolymer gives better processing than if pure polypropylene is substituted for the blend (Example 11) because the casting of Example 11 would not stretch to form film at 140°C. Example 12 shows that high calcium stearate levels greatly improves processibility compared to a low calcium stearate level in Example 13 because the resulting film had large visible pin holes and was extremely rough. Example 14 demonstrates a breathable composition composed of polypropylene and glass bead filler.

**Claims**

1. A method for making soft, flexible, microporous films having high tensile strength and good permeance or "breathability" levels for air and water vapor and high hydrostatic resistance to penetration by liquid water, including the steps of melt-blending a mixture of a polymer or copolymer of an alpha-olefin, a particulate filler, and calcium stearate as a processing aid, fabricating a film, and biaxially stretching the film, characterized in that the mixture contains 60 to 75% by weight of an inorganic filler or glass beads having particle sizes within the range of 10 to 15 micrometers in mean diameter, the moisture level in the blended composition is maintained below 700 parts per million prior to fabricating the film, and the film is stretched in two directions from about 1.5 to about 7 times in each direction at a temperature range of from about 20° to about 160°C.

2. A method for making microporous films as claimed in claim 1, further characterized in that the particle size of the filler is from 10 to 25 micrometers in mean diameter.

3. A method for making microporous films as claimed in claim 1 or 2, further characterized in that the filler is calcium carbonate and the particle size is about 12.5 micrometers in mean diameter.

4. A method for making microporous films as claimed in claim 1, 2, or 3, further characterized in that the moisture level in the blended composition is maintained below 300 parts per million prior to fabricating the film.

5. A method for making microporous films as claimed in any of the preceeding claims, further characterized in that the blended and extruded composition is cooled and its moisture level is simultaneously maintained by flowing air over it on a moving conveyor belt.

6. A method for making microporous films as claimed in any of the preceeding claims, further characterized in that the polymer or copolymer of an alpha-olefin is an alpha-olefin having 1-8 carbon atoms or a copolymer of ethylene with propylene or with an alpha-olefin of 4-8 carbon atoms.

7. A method for making microporous films as claimed in claim 6, further characterized in that the polymer or copolymer of an alpha-olefin is polypropylene, polyethylene, polybutylene, a blend of linear low density polyethylene and polypropylene, or a blend of polypropylene with ethylene-propylene copolymer.

8. A method for making microporous films as claimed in claim 7, further characterized in that the polymer or copolymer of an alpha-olefin is linear low-density polyethylene.

9. A-method for making microporous films as claimed in any of the preceeding claims, further characterized in that the mixture contains about 20 to 37% by weight of polypropylene, and the filler is calcium carbonate.

10. A method for making microporous films as claimed in claim 9, further characterized in that the mixture contains about 33% by weight of polypropylene, about 65% by weight of calcium carbonate, about 0.5 to 2% by weight of calcium stearate, and the casting is stretched in two directions 5 to 7 times at a temperature of about to 150 to 130°C.

11. A method for making microporous films as claimed in claim 9, further characterized in that the mixture contains about 2% by weight of calcium stearate, the moisture level is maintained below 300 ppm, and the casting is stretched in two directions 5 times in each direction at a temperature of about 130°C.

12. A method for making microporous films as claimed in any of the preceeding claims, further characterized in that the mixture contains about 20 to 37% by weight of polypropylene, and the filler is glass beads.

13. A method for making microporous films as claimed in claim 12, further characterized in that the mixture contains about 33.5% by weight of polypropylene, about 65% by weight of glass beads, about 0.5 to 2% by weight of calcium stearate, and the casting is stretched in two directions 5 to 7 times at a temperature of about to 130 to 150°C.

14. A method for making microporous films as claimed in claim 13, further characterized in that the mixture contains about 2% by weight of calcium stearate, the moisture level is maintained below 300 ppm, and the casting is stretched in two directions 5 times in each direction at a temperature of about 130°C.

15. A method for making microporous films as claimed in any of the preceeding claims, further characterized in that the mixture contains about 65 to 75% by weight of polybutylene, and the filler is calcium carbonate.

16. A method for making microporous films as claimed in claim 15, further characterized in that the mixture contains about 28% by weight of polybutylene, about 70% by weight of calcium carbonate, about 0.2 to 4% by weight of calcium stearate, and the casting is stretched in two directions 1.5 to 5 times at a temperature of about to 20 to 105°C.

17. A method for making microporous films as claimed in claim 16, further characterized in that the mixture contains about 2% by weight of calcium stearate, the moisture level is maintained below 300 ppm, and the casting is stretched in two directions 4 times in each direction at a temperature of about 100°C.

18. A method for making microporous films as claimed in any of the preceeding claims, further characterized in that the mixture contains about 60 to 70% by weight of polyethylene, and the filler is calcium carbonate.

19. A method for making microporous films as claimed in claim 18, further characterized in that the mixture contains about 28% by weight of polyethylene, about 70% by weight of calcium carbonate, about 0.1 to 3.5% by weight of calcium stearate, and the casting is stretched in two directions 1.5 to 5 times at a temperature of about to 20 to 105° C.

20. A method for making microporous films as claimed in claim 19, further characterized in that the mixture contains about 0.5 to 2% by weight of calcium stearate, the moisture level is maintained below 300 ppm, and the casting is stretched in two directions 4 times in each direction at a temperature of about 100° C.

21. A method for making microporous films as claimed in any of the preceeding claims, further characterized in that the mixture contains from about 0.1 to about 2% by weight of a stabilizer against degradation by exposure to UV light, oxygen, and heat.

22. A biaxially oriented microporous film comprising a polymer or copolymer of an alpha-olefin, a particulate filler, and calcium stearate, characterized in that it is made by the method as claimed in any of the preceeding claims and further characterized in that it has a Gurley porosity, based on method B, ASTM D-726, of 0.1 second to 20 seconds so that the film has good air and water vapor transmission rates but is substantially impenetrable by liquid water.

23. Use of the microporous film as claimed in claim 22, as a cover sheet for panty liners or a diaper.

24. Use of the microporous film as claimed in claim 22, as a bed sheet or a hospital gown.

25. Use of the microporous film as claimed in claim 22, as an ion-permeable separator in a liquid-containing battery.